**Europäisches Patentamt**

**European Patent Office**

**Office européen des brevets**

(19)

(11) Numéro de publication : **0 359 672 B1**

# FASCICULE DE BREVET EUROPEEN

(12)

(45) Date de publication du fascicule du brevet :
**19.11.92 Bulletin 92/47**

(51) Int. Cl.$^5$ : **A61F 2/36**

(21) Numéro de dépôt : **89402539.4**

(22) Date de dépôt : **15.09.89**

(54) **Prothèse articulaire, notamment prothèse fémorale, à effet d'auto-amortissement.**

(30) Priorité : **16.09.88 FR 8812135**

(43) Date de publication de la demande :
**21.03.90 Bulletin 90/12**

(45) Mention de la délivrance du brevet :
**19.11.92 Bulletin 92/47**

(84) Etats contractants désignés :
**AT BE CH DE ES GB GR IT LI LU NL SE**

(56) Documents cités :
**EP-A- 0 220 803**
**WO-A-86/06954**
**FR-A- 2 305 961**
**FR-A- 2 483 218**
**US-A- 4 642 124**

(73) Titulaire : **Bréard, Francis Henri**
**13, rue Friant**
**F-75014 Paris (FR)**

(72) Inventeur : **Bréard, Francis Henri**
**13, rue Friant**
**F-75014 Paris (FR)**

EP 0 359 672 B1

Jouve, 18, rue Saint-Denis, 75001 PARIS

# Description

La présente invention concerne une prothèse articulaire formée d'un corps allongé implantable par une extrémité dans le canal médullaire d'un os et recevant sur son autre extrémité une tête sphérique.

Les prothèses articulaires les plus répandues sont les prothèses fémorales dont la tête sphérique permet de reconstituer la forme naturelle de l'articulation du fémur, à l'extrémité d'un corps massif scellé dans le canal médullaire du fémur à l'aide d'un ciment spécial.

Il n'est pas rare toutefois que, sous l'effet des mouvements répétés de la jambe, les secousses ou autres sollicitations subies par la tête sphérique et transmises au corps allongé provoquent une fissuration du ciment et donc un descellement de la prothèse. Il en résulte des douleurs aiguës de la cuisse que l'on ne peut supprimer qu'en recimentant le corps de la prothèse dans le fémur, ce qui impose de pratiquer une nouvelle intervention chirurgicale sur le patient.

Pour pallier à ce fait, certains cherchent à transférer les chocs de la tête à la tige implantée par l'intermédiaire d'une articulation. Mais toute articulation finie par se casser. Une prothèse articulaire selon le préambule de la revendication 1 est décrit dans FR-A-23 05 961.

La présente invention se propose de remédier à cet inconvénient et, pour ce faire, elle a pour objet une prothèse articulaire du type précité qui se caractérise en ce que son corps est subdivisé, dans le sens de sa longueur, en une partie de support de la tête sphérique et une partie d'ancrage dans l'os, réunies l'une à l'autre par une liaison élastique amortisseuse.

Cette liaison amortisseuse sera de préférence réalisée par une liaison directe des parties de support et d'ancrage, au niveau d'une branche de jonction déportée vers la face externe du corps de la prothèse et maintenant ladite partie de support à l'aplomb de ladite partie d'ancrage, en lui conférant une possibilité de léger fléchissement ou débattement vers la face interne du corps, les faces externe et interne du corps de la prothèse étant ici définies comme étant celles qui sont tournées respectivement vers le côté externe et le côté interne de la cuisse une fois la prothèse implantée dans le fémur.

La prothèse selon l'invention est ainsi dotée d'un pouvoir d'auto-amortissement qui met la partie d'ancrage à l'abri des secousses ou autres sollicitations auxquelles peut être soumise la tête sphérique.

Dans ces conditions, le scellement en ciment réalisé autour de la partie d'ancrage pour assujettir le corps de la prothèse à l'os du fémur, ne sera pas atteint par ces secousses et conservera son intégrité. Les risques de descellement de la prothèse et d'apparition de douleurs de cuisse sont donc écartés ou tout au moins réduits dans une très large mesure.

Selon un premier mode de réalisation de l'invention, la branche de jonction desdites parties de support et d'ancrage est formée entre la face externe du corps et le fond d'une fente ménagée dans ce dernier depuis sa face interne, cette fente définissant lesdites parties de support et d'ancrage.

Dans une prothèse, notamment une prothèse fémorale, dont le corps est formé d'une tige intramédullaire surmontée d'un col qui reçoit à son extrémité libre la tête sphérique, la fente est avantageusement ménagée dans le tronçon épiphysaire de la tige intramédullaire.

De préférence, cette fente est située dans un plan sensiblement perpendiculaire à l'axe du col et son fond s'élargit sous une forme arrondie, ces deux dernières caractéristiques ayant pour résultat de procurer le meilleur effet d'amortissement possible.

Selon une autre caractéristique de l'invention, la fente s'étend sur une profondeur telle que la largeur qu'elle détermine pour ladite zone de jonction, soit approximativement égale à la hauteur qu'a la partie de support dans un plan sensiblement perpendiculaire au plan de la fente, au droit du fond de cette dernière. Il s'agit là du compromis optimal entre la dimension et le positionnement de la fente pour supprimer tout risque de rupture de la partie de support au niveau de sa zone de jonction avec la partie d'ancrage.

On peut additionnellement remplir la fente, au moins partiellement du côté de son ouverture, d'un matériau compressible, évitant l'introduction de ciment dans la fente.

Selon un deuxième mode de réalisation d'une prothèse selon l'invention, dont le corps est également formé d'une tige intramédullaire, surmontée d'un col qui reçoit à son extrémité libre la tête sphérique, la partie d'ancrage est constituée par la totalité de la tige intramédullaire et la partie de support de la tête sphérique est formée par le col qui, par son pied constituant ladite branche de jonction, se rattache à la tige du côté de la face externe de cette dernière et s'incline vers sa face interne.

Tout en conservant sa fonction classique de support de la tête sphérique sur le côté intérieur de la prothèse, le col peut légèrement fléchir lorsque cette dernière est sollicitée, et jouer donc à lui seul le rôle de l'élément amortisseur de la prothèse.

Pour éviter tout risque de rupture de ce col au niveau de sa liaison avec la tige, il sera préférable de le réaliser sous la forme d'un élément rapporté dont le matériau constitutif présente une élasticité propre.

En variante ou en complément, on peut toutefois obtenir le même résultat, dans une prothèse conforme à un troisième mode de réalisation de l'invention, en donnant au col une forme telle qu'avec le fond d'un évidement ménagé dans la face en regard de la tige intramédullaire, il délimite une cavité arrondie débouchant sur la face interne du corps de la prothèse et s'ouvrant sur les faces antérieure et postérieure de celui-ci.

De préférence, l'ouverture par laquelle cette cavité débouche sur la face externe du corps de la prothèse est délimitée par deux butées saillant l'une en face de l'autre, respectivement sur le col et sur la tige. Ces butées ont pour fonction, dans les cas de sollicitations extrêmes de la tête sphérique, de limiter l'amplitude de fléchissement du col pour le mettre à l'abri d'une rupture au niveau de sa zone de jonction avec la tige.

La présente invention va maintenant être décrite plus en détails, mais sans caractère limitatif, en référence aux dessins annexés dans lesquels :
- la figure 1 est une vue en élévation latérale d'une prothèse conforme au premier mode de réalisation de l'invention ;
- la figure 2 représente la partie supérieure de la prothèse de la figure 1;
- la figure 3 est une vue en coupe agrandie selon la ligne III-III de la figure 1 ;
- la figure 4 est une vue en élévation de la partie supérieure d'une prothèse selon le deuxième mode de réalisation de l'invention ;
- la figure 5 est une vue en élévation d'une prothèse selon le troisième mode de réalisation de l'invention ;
- la figure 6 est une vue agrandie de la partie supérieure de la prothèse de la figure 5 et ;
- la figure 7 est une vue en coupe effectuée dans le plan VII-VII de la figure 4.

Dans ses trois modes de réalisation exemplifiés, la prothèse selon l'invention, en l'occurrence une prothèse fémorale, comprend une tige intramédullaire, désignée dans son ensemble par 1, 1a ou 1b, qui est surmontée au-delà de sa face supérieure élargie 2, 2a, par un col 4, 4a ou 4b portant à son extrémité libre une tête 3 de forme générale sphérique.

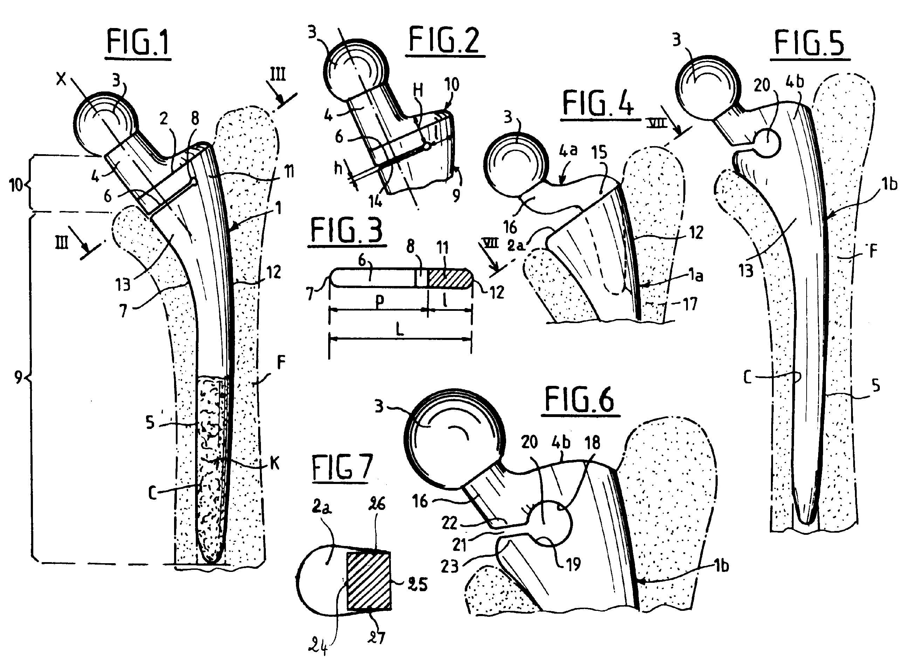

La tige intramédullaire présente, à son extrémité opposée au col, une partie sensiblement cylindrique 5 à diamètre égal au diamètre du canal médullaire C du fémur F, par laquelle elle peut être implantée dans ce dernier et y être ancrée à l'aide d'un scellement en ciment, tel que représenté partiellement en K sur la figure 1 uniquement. Entre cette partie cylindrique 5 et la face supérieure 2 de la tige 1, 1a, 1b, s'étend le tronçon épiphysaire élargi 13 de cette dernière, qui est reçu dans la partie haute évasée du canal médullaire C.

Dans le premier mode de réalisation de l'invention représenté sur la figure 1, une fente continue 6 a été pratiquée, par exemple par un simple trait de scie, dans le tronçon épiphysaire 13 de la tige 1, non loin de sa face supérieure 2. Cette fente 6, qui s'ouvre sur les larges faces antérieures et postérieures de la tige 1, débouche par ailleurs sur la tranche ou face interne 7 de la tige 1, c'est-à-dire celle qui est tournée vers l'aine une fois la prothèse implantée dans la cuisse du patient, et se termine de l'autre côté par un fond 8 qui s'élargit sous une forme arrondie. La fente 6 s'étend par ailleurs dans un plan sensiblement perpendiculaire à l'axe X du col cylindrique 4, lequel est positionné, comme dans les prothèses fémorales classiques, sur le côté interne de la prothèse.

La fente 6 subdivise ainsi la prothèse en deux parties, à savoir une longue partie d'ancrage 9, comprenant la partie d'extrémité cylindrique 5 et la portion inférieure du tronçon épiphysaire 13 de la tige 1, et une partie de support 10 de la tête sphérique, constituée de la courte portion supérieure du tronçon épiphysaire et du col 4. La partie de support 10 est, du côté interne de la prothèse, maintenue à l'aplomb ou en porte-à-faux au-dessus de la partie d'ancrage 9 par une courte branche de jonction 11 qui réunit ces deux parties l'une à l'autre entre le fond 8 de la fente 6 et la tranche externe 12 de la tige intramédullaire 1.

Cette branche de jonction 11, qui est venue de matière avec les parties de support 10 et d'ancrage 9 de la prothèse généralement réalisée en titane ou un alliage à base de titane, constitue une liaison élastique qui autorise un léger fléchissement, vers l'intérieur, de la première par rapport à la seconde lorsque la tête sphérique est soumise à des secousses ou autres sollicitations.

La prothèse selon l'invention dispose ainsi d'un pouvoir d'auto-amortissement qui isole le scellement K en ciment, présent uniquement autour de la partie d'ancrage 9, des secousses ou autres efforts s'exerçant sur la tête sphérique et le préserve ainsi de toute fissuration ou autre dégradation. Pour éviter toute pénétration de ciment dans la fente 6, on pourra remplir cette dernière d'une résine compressible, comme indiqué en 14, sur la figure 2.

La figure 2 montre en outre que la largeur l de cette branche de jonction 11, c'est-à-dire sa dimension mesurée entre la tranche externe 12 de la tige 1 et le fond 8 de la fente 6 est sensiblement égale à la hauteur H qu'a la partie de support 10 au droit du fond 8 de la fente, dans un plan perpendiculaire à cette dernière. Cette condition détermine le positionnement et la profondeur p de la fente 6 qui, dans l'exemple représenté, correspond approximativement aux 2/3 de la largeur L qu'a la tige 1 dans le même plan (voir figure 3). La fente 6 a par ailleurs une hauteur h d'environ 1 à 2 mm.

Ces caractéristiques de dimensionnement et de positionnement de la fente sont celles qui optimiseront la résistance à la rupture de la partie de support 10 au niveau de la branche de jonction 11.

Dans le deuxième mode de réalisation de l'invention représenté sur la figure 4, la tige intramédullaire 1a de la prothèse est une tige classique massive, alors que le col 4a est relié à la tige 1a non pas du côté interne de la prothèse, mais à proximité de sa tranche externe 12 par un pied élargi 15 couvrant environ la moitié externe de la section de la tige. Il s'incline en outre vers l'intérieur de la prothèse sous un angle d'environ 45° pour se terminer à son extrémité libre par une partie 16 recourbée vers le haut qui maintient la tête sphérique dans la position qu'elle a sur la prothèse de la figure 1. Le col 4a a en fait la forme générale d'un col de cygne.

Le col 4, grâce à cette inclinaison, peut légèrement fléchir vers l'intérieur lorsque la tête 3 est soumise à des efforts et joue ainsi à lui seul le rôle de l'élément amortisseur de la prothèse, rempli dans le mode de réalisation de la figure 1 par la partie de support 10.

Pour bénéficier d'une protection optimale contre les risques de rupture, le col 4a sera de préférence réalisé en un matériau composite, présentant une élasticité propre, et rapporté sur la tige 1a comme illustré en traits interrompus en 17.

Les figures 5 et 6 montrent un troisième mode de réalisation de la prothèse selon l'invention, dont le col 4b, venu de matière avec la tige métallique 1b ou rapporté sur celle-ci, présente la même forme générale que celui 4a du mode de réalisation de la figure 4, à la différence près qu'il est légèrement creusé, comme indiqué en 18, en regard du sommet de la tige 1b. Dans cette dernière est par ailleurs ménagé un évidement 19 sensiblement semicirculaire et situé exactement en face du creux 16 du col 4b pour définir, sans discontinuité, avec celui-ci une cavité 20 de forme générale circulaire.

Cette cavité 20, qui s'ouvre en 21 sur la face interne de la prothèse, confère au col 4b une possibilité de léger fléchissement élastique tout en renforçant sa résistance à la rupture au niveau de sa liaison avec la tige 1b.

On peut encore voir sur la figure 6 que l'ouverture 21 de la cavité 20 est une fente d'une hauteur de 2 à 3 mm, délimitée par des butées 22, 23 en regard mutuel, formées en léger relief, l'une 22 sous la partie d'extrémité 16 du col 4b et l'autre 23 sur la pointe corticale de la tige 1b. Ces deux butées 22, 23 ont pour utilité de limiter, par contact mutuel, l'amplitude de fléchissement du col et par conséquent de prévenir une rupture de ce dernier, dans des cas très occasionnels, en particulier à la suite d'un effort excessif exercé sur la tête sphérique 3.

On précisera encore, en référence à la figure 7, que, dans le mode de réalisation représenté sur la figure 4, le col 4a de la prothèse est doté d'une section rectangulaire, avec ses grands côtés ou plats 24, 25 tournés vers les faces interne 7 et externe 12 de la prothèse et ses petits côtés ou chants 26, 27 dirigés vers les faces antérieure et postérieure de celle-ci. Cette caractéristique de forme, qui peut également se retrouver dans le mode de réalisation des figures 5 et 6, renforce l'élasticité du col de la prothèse.

## Revendications

1. Prothèse articulaire, notamment prothèse fémorale, constituée d'un corps allongé subdivisé, dans le sens de sa longueur, en une partie d'ancrage (9 ; 1a ; 1b) implantable par une extrémité (5) dans le canal médullaire (C) d'un os et une partie de support (10 ; 4a ; 4b) d'une tête sphérique (3), réunie à l'autre extrémité de la partie d'ancrage par une liaison amortisseuse, caractérisée en ce que la liaison amortisseuse entre la partie de support (10 ; 4a ; 4b) et la partie d'ancrage (9 ; 1a ; 1b) est réalisée par une branche de jonction de ces dernières, déportée vers la face externe (12) du corps, venue de matière avec l'une au moins desdites parties de support et d'ancrage et maintenant la partie de support (10 ; 4a ; 4b) à l'aplomb de la partie d'ancrage (9 ; 1a ; 1b), avec un jeu de débattement entre elles.

2. Prothèse selon la revendication 1, caractérisée en ce que ladite branche de jonction (11) est formée entre la face externe (12) du corps et le fond (8) d'une fente (6) ménagée dans ce dernier depuis sa face interne (7) et définissant lesdites parties de support (10) et d'ancrage (9).

3. Prothèse selon la revendication 2, caractérisée en ce que le corps de la prothèse est formé d'une tige intramédullaire (1) surmontée d'un col (4) qui reçoit à son extrémité libre la tête sphérique (3), et que la fente (6) est ménagée dans le tronçon épiphysaire (13) de la tige intramédullaire (1).

4. Prothèse selon la revendication 3, caractérisée en ce que la fente (6) est située dans un plan sensiblement perpendiculaire à l'axe (X) du col (4).

5. Prothèse selon les revendications 2, 3 ou 4, caractérisée en ce que la fente (6) s'étend sur une profondeur (p) telle que la largeur (1) qu'elle détermine pour ladite zone de jonction (11), soit approximativement égale à la hauteur (H) qu'à la partie de support dans un plan sensiblement perpendiculaire au plan de la fente (6), au droit du fond (8) de cette dernière.

6. Prothèse selon l'une quelconque des revendications 2 à 5, caractérisée en ce que le fond (8) de la fente (6) s'élargit sous une forme arrondie.

7. Prothèse selon l'une quelconque ces revendications 2 à 6, caractérisée que la fente (6) est au moins du côté de son ouverture sur la face interne du corps, remplie d'un matériau compressible (14).

8. Prothèse selon la revendication 1, caractérisée en ce que le corps de la prothèse est formé d'une tige intramédullaire (1a ; 1b) surmontée d'un col (14a ; 4b) qui reçoit à son extrémité libre la tête sphérique (3), et que la partie d'ancrage est constituée par la totalité de la tige intramédullaire (1a ; 1b) et la partie de support de la tête sphérique est formée par le col (4a ; 4b) qui, par son pied constituant ladite branche de jonction, se rattache à la tige du côté de la face externe (12) de cette dernière et s'incline vers sa face interne (7).

9. Prothèse selon la revendication 8, caractérisée en ce que le col (4a) est un élément rapporté, réalisé en un matériau présentant une élasticité propre.

10. Prothèse selon la revendication 8 ou 9, caractérisée en ce que le col (4b) délimite avec le fond d'un évidement (19) formé dans la face en regard de la tige, une cavité arrondie (20) débouchant sur la face interne du corps de la prothèse.

11. Prothèse selon la revendication 10, caractérisée en ce que l'ouverture (21) par laquelle ladite cavité (20) débouche sur la face interne (7) du corps de la prothèse est délimitée par deux butées (22, 23) saillant l'une en face de l'autre, respectivement sur le col (4b) et sur la tige (1b).

12. Prothèse selon l'une des revendications 8 à 11, caractérisée en ce que le col présente, en section transversale, la forme d'un rectangle dont les grands côtés sont tournés respectivement vers la face interne (7) et la face externe (12) de la prothèse.

**Patentansprüche**

1. Gelenkprothese, insonderheit Hüftkopfersatz, die aus einem länglichen Körper besteht, der der Länge nach in einen Verankerungsteil (9; 1a; 1b), der an einem Ende (5) in den Markkanal eines Knochens implantiert wird, und in einen Halterungsteil (10; 4a; 4b) für einen spherischen Kopf (3) unterteilt ist, der mit dem anderen Ende des Verankerungsteiles durch eine dämpfende Verbindung verbunden ist; die Prothese ist dadurch gekennzeichnet ist, dass diese dämpfende Verbindung zwischen dem Halterungsteil (10; 4a; 4b) und dem Verankerungsteil (9; 1a; 1b) aus einem Verbindungsarm dieser Teile besteht, der zur Aussenseite (12) des Körpers verschoben ist und der mit zumindest einem der beiden Teile - Halterung- oder Verankerungsteil - in Berührung ist und der den Halterungsteil (10; 4a; 4b) lotrecht zum Verankerungsteil (9; 1a; 1b) mit einem Ausschlagsspiel zwischen beiden Teilen hält.

2. Prothese laut Patentanspruch 1, die dadurch gekennzeichnet ist, dass der Verbindungsarm (11) zwischen der Aussenseite (12) des Körpers und dem Boden (8) aus einem Schlitz (6) besteht, mit dem der Körper von der Innenseite (7) her versehen ist, und durch den die Halterungs- (10) und Verankerungsteile (9) definiert werden.

3. Prothese laut Patentanspruch 2, die dadurch gekennzeichnet ist, dass der Prothesenkörper aus einem intramedullarem Stift (1) besteht, auf dem ein Hals (4) sitzt, auf dessen freiem Ende wiederum der spherische Kopf (3) angebracht ist und die dadurch gekennzeichnet ist, dass sich der Schlitz (6) im epiphysären Abschnitt (13) des intramedullaren Stiftes (1) befindet.

4. Prothese laut Patentanspruch 3, die dadurch gekennzeichnet ist, dass der Schlitz (6) leicht senkrecht zur Achse (X) des Halses (4) ist.

5. Prothese laut Patentanspruch 2, 3 oder 4, die dadurch gekennzeichnet ist, dass sich der Schlitz (6) über eine Tiefe (p) so weit erstreckt, dass die Breite (1), die er für die besagte Verbindungszone (11) definiert ungefähr die gleiche Höhe (h) aufweist, wie der Halterungsteil in leicht senkrechter Ebene zur Ebene des Schlitzes (6), an der Stelle, wo sich der Boden (8) desselben befindet.

6. Prothese laut eines beliebigen Patentanspruches zwischen 2 und 5, die dadurch gekennzeichnet ist, dass sich hder Boden (8) des Schlitzes (6) in gerundeter Form erweitert.

7. Prothese laut eines beliebigen Patentanspruches zwischen 2 und 6, die dadurch gekennzeichnet ist, dass der Schlitz mindestens an der Seite, an der er sich zur Innenseite des Körpers hin öffnet mit einem kompressiblen Werkstoff (14) ausgefüllt ist.

8. Prothese laut Patentanspruch 1, die dadurch gekennzeichnet ist, dass der Prothesenkörper aus einem intramedullaren Stift (1a; 1b) gebildet wird, auf dem ein Hals (4a; 4b) sitzt, auf dessen freiem Ende der spherische Kopf (3) angebracht ist und die dadurch gekennzeichnet ist, dass der Verankerungsteil aus dem gesamten intramedullaren Stift (1a; 1b) und der Halterungsteil des spherischen Kopfes durch den Hals (4a; 4b) gebildet wird, der mit seinem Fuss, der den besagten Verbindungsarm bildet, mit dem Stift an seiner Aussenseite (12) verbunden ist und sich zu seiner Innenseite (7) neigt.

9. Prothese laut Patentanspruch 8, die dadurch gekennzeichnet ist, dass der Hals (4a) aus einem aufgesetzten Element besteht, das aus einem Werkstoff mit Eigenelastizität gefertigt ist.

10. Prothese laut Patentanspruch 8 oder 9, die dadurch gekennzeichnet ist, dass der Hals (4b) mit dem Boden einer Aushöhlung (19), die in der Seite gegenüber dem Stift eingelassen ist, eine gerundete Höhlung (20) begrenzt, die zur Innenseite des Prothesenkörpers weist.

11. Prothese laut Patentanspruch 10, die dadurch gekennzeichnet ist, dass die Öffnung (21), durch die die Höhlung (20) auf die Innenseite (7) des Prothesenkörpers weist, über zwei Begrenzungen (22, 23) begrenzt wird, die sich gegenüberstehen, wobei sich eine am Hals (4b) und eine am Stift (1b) befindet.

12. Prothese laut Patentanspruch 8 bis 11, die dadurch gekennzeichnet ist, dass der Hals im Querschnitt die Form eines Rechteckes aufweist, von dessen langen Seiten eine zur Innenseite (7) und eine zur Aussenseite (12) der Prothese gedreht ist.

## Claims

1. Articular prosthesis, notably femoral prosthesis, constituted of an elongated body subdivided along its length into an anchoring section (9; 1a; 1b) implantable by one extremity (5) in the medullary cavity (C) of a bone and a support section (10; 4a; 4b) with a spherical head (3), joined to the other extremity of the anchoring section by a damping linkage, characterized in that the damping linkage between the support section (10; 4a; 4b) and the anchoring section (9; 1a; 1b) is produced by a point of junction of these latter, offset towards the external face (12) of the body, contiguous with at least one of said support or anchoring sections, and maintaining the support section (10; 4a; 4b) perpendicular to the anchoring section (9; 1a; 1b), with a free clearance between them.

2. Prosthesis according to claim 1, characterized in that said point of junction (11) is formed between the external face (12) of the body and the endpoint (8) of a slit (6) cut into the latter from its internal face (7) and defining said support (10) and anchoring (9) sections.

3. Prosthesis according to claim 2, characterized in that the body of the prosthesis is formed from an intramedullary shaft (1) topped by a neck (4) which at its free end receives the spherical head (3), and that the slit (6) is cut into the epiphyseal stump (13) of the intramedullary shaft (1).

4. Prosthesis according to claim 3, characterized in that the slit (6) is located in a plane approximately perpendicular to the axis (X) of the neck (4).

5. Prosthesis according to claims 2, 3 or 4, characterized in that the slit (6) extends over a depth (p) such that the width (1) that it determines for said junction region (11) is approximately equal to the height (H) of the support section in a plane approximately perpendicular to the plane of the slit (6), to the right of the endpoint (8) of this latter.

6. Prosthesis according to any one of claims 2 through 5, characterized in that the endpoint (8) of the slit (6) widens into a rounded shape.

7. Prosthesis according to any one of claims 2 through 6, characterized in that the slit (6) is at least from the side of its opening on the internal face of the body, filled with a compressible material (14).

8. Prosthesis according to claim 1, characterized in that the body of the prosthesis is formed from an intramedullary shaft (1a; 1b) topped by a neck (4a; 4b) which at its free end receives the spherical head (3) and that the anchoring section is constituted by the entire intramedullary shaft (1a; 1b) and the support section of the spherical head is formed by the neck (4a; 4b) which, by its foot constituting said junction point, joins the shaft from the side of the external face (12) of the latter and inclines towards its internal face (7).

9. Prosthesis according to claim 8, characterized in that the neck (4a) is an add-on unit made from a material with suitable elasticity.

10. Prosthesis according to claim 8 or 9, characterized in that the neck (4b) demarcates, with the bottom of a recess (19) formed in the face of the shaft, a rounded cavity (20) opening into the internal face of the body of the prosthesis.

11. Prosthesis according to claim 10, characterized in that the opening (21) by which said cavity (20) opens into the internal face (7) of the body of the prosthesis is demarcated by two abutments (22, 23), one opposite the other, protruding respectively on the neck (4b) and on the shaft (1b).

12. Prosthesis according to one of claims 8 through 11, characterized in that the neck has, in cross section, the form of a rectangle whose longer sides are respectively turned towards the internal face (7) and the external face (12) of the prosthesis.

FIG.1

FIG.2

FIG.3

FIG.4

FIG.5

FIG.6

FIG.7

EP 0 359 672 B1